# EUROPÄISCHE PATENTSCHRIFT

(11) **EP 3 297 762 B1**
(45) Veröffentlichungstag und Bekanntmachung des Hinweises auf die Patenterteilung: **11.12.2019**
(21) Anmeldenummer: 16721823.9
(22) Anmeldetag: 11.05.2016
(51) Int. Cl.: B05B 1/14, B65D 83/20, B65D 83/30, B65D 83/14, A61M 15/00, A61M 11/00, B65D 83/24

(54) **INHALATIONSEINRICHTUNG UND INHALATIONSEINRICHTUNGS-SET**
INHALATION DEVICE AND INHALATION DEVICE SET
DISPOSITIF D'INHALATION ET KIT POUR UN DISPOSITIF D'INHALATION

(30) Priorität: 20.05.2015 EP 15168316
(43) Veröffentlichungstag der Anmeldung: 28.03.2018
(73) Patentinhaber: Aptar Radolfzell GmbH, 78315 Radolfzell (DE)
(72) Erfinder: NADLER, Günter, 78345 Moos-Iznang (DE)
(74) Vertreter: Patentanwaltskanzlei Cartagena
(86) Internationale Anmeldenummer: PCT/EP2016/060605
(87) Internationale Veröffentlichungsnummer: WO 2016/184761

(56) Entgegenhaltungen:
- EP-A1- 1 698 399
- EP-A1- 1 792 660
- WO-A1-2011/147714
- WO-A1-2013/064690
- WO-A2-2015/149922
- DE-U1- 8 906 590
- DE-U1- 8 906 590
- GB-A- 2 202 591
- US-A- 3 186 407
- US-B1- 6 338 442

## Beschreibung

Die Erfindung betrifft eine Inhalationseinrichtung zum Inhalieren einer Flüssigkeit in vernebelter Form nach Anspruch 1.

Die Erfindung betrifft weiterhin ein Inhalationseinrichtungs-Set nach dem Oberbegriff von Anspruch 13. Inhalationseinrichtungen zum Inhalieren einer Flüssigkeit in vernebelter Form sind bekannt.

Solche Inhalationseinrichtungen dienen dem Zweck, Flüssigkeit zu vernebeln, so dass dieses durch Einatmen in die Atemwege, die Bronchien und die Lunge eines Patienten gelangen kann. Neben klassischen Medikamenten finden solche Inhalationseinrichtungen insbesondere auch Anwendung zum Austrag von Salzwasser oder anderweitig mit ätherischen Ölen ergänztem Wasser. Hierdurch wird an Erkältung und Grippe Erkrankten eine einfache Möglichkeit gegeben, die Atmung wieder zu erleichtern und Schmerzen im Nasen-, Hals- und Rachenraum sowie in der Lunge mit einfachen Mitteln zu lindern.

Bekannte Inhalationseinrichtungen sind meist entweder sehr einfach aufgebaut, indem sie nicht sehr viel mehr als eine mit einem Speicher verbundene Atemmaske zur Verfügung stellen, wobei der Speicher für jede Anwendung neu zu befüllen ist, oder sie sind eher komplex aufgebaut sind und zum Teil mit einer Stromversorgung versehen sind, um die Vernebelung des Fluids zu gestatten. Solche letztgenannten Vorrichtungen sind häufig nicht ohne weiteres transportabel.

DE 8906590 und EP 1792660 A1 zeigen Inhalationseinrichtungen, die über einen Applikatorstück verfügen, das seitlich an einem Applicatorkopf vorgesehen ist.

EP 1237610 zeigt eine Inhalationseinrichtung, die über ein schaltbares Dreh-Auslassventil verfügt.

### AUFGABE UND LÖSUNG

Aufgabe der Erfindung ist es, eine Inhalationseinrichtung zur Verfügung zu stellen, die gegenüber den bekannten Inhalationseinrichtungen in Hinblick auf Transportabilität, einen einfachen Aufbau, eine bequeme Bedienung und/oder geringe Herstellungskosten von Vorteil ist.

Die Aufgabe wird durch eine Inhalationseinrichtung zum Inhalieren einer Flüssigkeit in vernebelter Form nach Anspruch 1 gelöst.

Die Inhalationseinrichtung gemäß Anspruch 1 verfügt über ein Gehäuse, das zumindest abschnittsweise als rotationssymmetrischer Körper ausgestaltet ist, dessen Mittelachse eine Haupterstreckungsachse des Gehäuses definiert, und welches einen Flüssigkeitsspeicher umgibt, in dem die Flüssigkeit vor dem Austrag durch Beaufschlagung mit Treibgas, mit Druckluft oder durch eine vorgespannte Federeinrichtung unter Druck stehend gelagert ist.

Die Inhalationseinrichtung verfügt über einen Applikatorkopf mit einem Zerstäubungsraum und einem mit dem Zerstäubungsraum verbundenen Applikatorstück, das seitlich am Applikatorkopf vorgesehen ist, wobei das Applikatorstück entweder als Mundstück zur Aufnahme im Mund eines Patienten oder als Inhalationsmaske zur dichten Überdeckung des Mundes, der Nase oder des Mundes und der Nase oder als Adapterstück zur Anbringung eines Mundstücks oder einer Inhalationsmaske ausgebildet ist.

Die Inhalationseinrichtung verfügt über einen Austragkanal, der den Flüssigkeitsspeicher mit dem Applikatorkopf verbindet, und über ein schaltbares Dreh-Auslassventil, durch das der Austragkanal geöffnet und geschlossen werden kann.

Das Dreh-Auslassventil ist durch eine Drehbewegung des Gehäuses um die Haupterstreckungsachse gegenüber dem Applikatorstück schaltbar.

Bei einer solchen Gestaltung, die auch über eine unten beschriebene Düsenplatte verfügen kann, jedoch nicht muss, ist eine besonders gute Bedienbarkeit dadurch gegeben, dass das Applikatorstück seitlich am Applikatorkopf und damit exzentrisch zur Mittelachse vorgesehen ist. Der Benutzer kann dadurch durch Anpressen an das Gesicht das Applikatorstück lagefixieren, so dass anschließend eine Drehbewegung des Schaltelements oder vor allem des Gehäuses zum Zwecke des Öffnens oder Schließens des Dreh-Auslassventils einfach und intuitiv möglich ist.

Eine durch das Applikatorstück definierte Austragrichtung ist hierfür vorzugsweise gegenüber der Haupterstreckungsachse des Gehäuses angewinkelt, wobei insbesondere vorzugsweise die Austragrichtung und die Haupterstreckungsachse einen Winkel zwischen 10° und 170° einschließen, insbesondere vorzugsweise einen Winkel zwischen 60° und 120°. Insbesondere kann ein rechter Winkel (90°) hier gewählt werden.

Vorzugsweise ist ein Lufteinlass vorgesehen, der in den Zerstäubungsraum mündet. Durch Einsaugen von Luft am Applikatorstück kann ein Nutzer Luft in den Zerstäubungsraum einsaugen, wo dieser mit zerstäubter Flüssigkeit versehen wird und dann durch das vorzugsweise gegenüberliegend angeordnete Applikatorstück abgegeben wird.

Dem Auslassventil kann eine Federeinrichtung zugeordnet sein, die derart ausgebildet ist, dass ihre Federkraft stets versucht, das Auslassventil zu schließen.

Eine solche Federeinrichtung kann unmittelbar Teil des Auslassventils sein. Die Federeinrichtung kann jedoch auch zwischen dem Gehäuse und dem Applikatorkopf bzw. dem Gehäuse und dem Applikatorstück vorgesehen sein und nur mittelbar auf das Ventil wirken.

Der Applikatorkopf kann über ein drehfest mit dem Gehäuse verbundenes Basisteil und über ein demgegenüber drehbares Auslassteil, welches den Zerstäubungsraum umfasst, verfügen. Der Applikatorkopf und das Auslassteil aneinander derart geführt sein, dass eine Drehbewegung des Auslassteils gegenüber dem Basisteil eine axiale Verlagerung der Auslassteils gegenüber dem Basisteil bewirkt, durch die ein Auslassventil der Inhalationseinrichtung schaltbar ist.

Dies gestattet eine besonders einfache Bauweise des Applikatorkopfes mit nur zwei relativbeweglichen Teilen und insgesamt mitsamt Düsenplatte nur drei Bauteilen. Die Basis wird am Gehäuse der Inhalationseinrichtung angebracht. Sie verfügt vorzugsweise über eine Führungsschräge, insbesondere als Teil eines Innengewindes oder einer Kulisse. Das zweite Teil, das Auslassteil, ist drehbar an der Basis angebracht, wobei es durch Führungsabschnitte an der Führungsschräge geführt ist, so dass die Drehbewegung auch eine axiale Verlagerung zur Folge hat. Diese axiale Verlagerung kann ein Ventil öffnen, welches im Gehäuse in der Nähe des Flüssigkeitsspeichers oder am Applikatorkopf vorgesehen sein kann. Das Auslassteil umfasst vorzugsweise sowohl die Zerstäubungskammer als auch einen darin mündenden Auslass mit Düsenplatte. Das Auslassteil kann daher sehr einfach als Ganzes unter dem Wasserhahn gereinigt werden.

Im Flüssigkeitsspeicher kann insbesondere eine wässrige Lösungen, vor allem eine salzhaltige wässrige Lösung enthalten. Weiterhin kann die Lösung eine gepufferte Lösung oder eine Ringerlösung darstellen. Es kann sich unmittelbar um Meerwasser handelt. Die wässrige Lösung kann mit verschiedenen Zusätzen versehen sein. Insbesondere können Kohlenhydrate, ätherische Öle, Menthol und Pflanzenextrakte enthalten sein. Auch enthalten sein können Vitamine und Spurenelemente sowie Mangan oder Zink. Auch Träger- oder Hilfsstoffe als Träger aktiver Substanzen können enthalten sein. Die Flüssigkeit im Flüssigkeitsspeicher kann insbesondere auch Zusätze aus der Gruppe umfassen Zimtöl, Teebaumöl, Salbeiöl, Thymianöl, Zitronenmelissenöl enthalten.

Der Austragkanal kann zumindest abschnittsweise mit einem antibakteriell wirkenden Material versehen sein, insbesondere mit Silber oder ein Silber beinhaltendes Material.

Die Verwendung eines antibakteriell wirkenden Materials im Austragkanal stromaufwärts der Düsenplatte hat sich als besonders vorteilhaft herausgestellt, da die Düsenplatte ein Trocknen des Austragkanals nach erfolgtem Austrag nur in geringem Maße gestattet. Die Verdunstung durch die dünnen Düsenöffnungen hindurch ist gering. Das antibakteriell wirkende Material kann entlang des Kanals an der Wandung aufgebracht sein. Denkbar ist auch eine poröse oder schwammartige Struktur aus antibakteriellem Material, die im Austragkanal angeordnet ist. Besonders bevorzugt wird es, wenn eine Innenseite der Düsenplatte antibakteriell ausgestaltet ist, beispielsweise durch eine Silberbeschichtung.

Das Applikatorstück und die Düsenplatte können Teil einer gemeinsamen Baueinheit sein, die werkzeuglos vom Gehäuse lösbar ist.

Die Vereinigung des Applikatorstücks und der Düsenplatte in einer gemeinsamen Baueinheit, die beispielsweise mittels Rastverbindungsmitteln einfach vom Gehäuse lösbar und wieder aufsetzbar ist, bietet den Vorteil separater Waschbarkeit einschließlich der Düsenplatte. Auch kann hierdurch in einfacher Weise ein Austausch dieser Baugruppe aus hygienischen Gründen erfolgen.

Die Aufgabe wird auch durch ein erfindungsgemäßes Inhalationseinrichtungs-Set gelöst.

Dieses Set umfasst eine Inhalationseinrichtung nach vorbeschriebener Art. Es umfasst mindestens zwei Baueinheiten, die jeweils ein Applikatorstück und eine Düsenplatte umfassen.

Ein solches Set gestattet es, ohne hygienische Bedenken die Inhalationseinrichtung durch mehrere Personen verwenden zu lassen, indem jede Person jeweils nur ihre eigene austauschbare Baueinheit verwendet. Da diese die größte Gefahr einer Kontamination birgt, wird Ansteckungsgefahr hierdurch wirksam vermieden.

Die Düsenplatte einer erfindungsgemäßen Inhalationseinrichtung umfasst eine Vielzahl von Düsenöffnungen. Die Düsenplatte ist vorzugsweise zumindest abschnittsweise aus Silber oder aus einem Silber beinhaltenden Material gebildet.

Es hat sich gezeigt, dass die Verwendung von Silber an einer Düsenplatte wirksam die Kontamination verhindert, insbesondere auch die Kontamination in den Düsenöffnungen selbst.

Die Düsenplatte kann Düsenöffnungen mit einem Durchmesser zwischen 1 µm und 100 µm, insbesondere zwischen 2 µm und 10 µm, umfassen. Die Düsenplatte kann mindestens 9 Düsenöffnungen, vorzugsweise mindestens 16 Düsenöffnungen und insbesondere vorzugsweise mindestens 25 Düsenöffnungen, umfassen.

Die Düsenplatte kann als Ganzes aus Silber oder einer Silberlegierung gefertigt sein. Dies wird jedoch aus wirtschaftlichen Erwägungen üblicherweise nicht als ideal angesehen.

Stattdessen kann die Düsenplatte eine Grundplatte aus einem Material aufweisen, welches kein Silber oder Silber beinhaltendes Material beinhaltet. Auf der Grundplatte kann eine Schicht aus Silber oder einem Silber beinhaltenden Material wie einer Silberlegierung vorgesehen sein.

Die Schicht aus Silber oder einem Silber beinhaltenden Material kann nach Einbringung der Düsenöffnungen durch Bedampfen oder Eintauchen auf die Grundplatte aufgebracht sein, so dass die Düsenöffnungen an ihren Innenseiten zumindest teilweise mit dieser Schicht bedeckt sind.

Die Grundplatte kann beispielsweise aus Silizium gefertigt sein, welches sich hierfür als sehr gut geeignetes Material herausgestellt hat. Das Silber oder das Silber enthaltende Material ist darauf in Form einer dünnen Schicht aufgebracht. Diese Schicht kann innenseitig und/oder außenseitig und/oder in den Düsenöffnungen selbst gegeben sein.

Die Düsenöffnungen können nach Bedampfen oder Eintauchen der Grundplatte zum Zwecke der Herstellung der Schicht aus Silber oder Silberoxid eingebracht sein, so dass die Düsenöffnungen an ihrer Innenseite zumindest teilweise frei von dieser Schicht sind.

Ein solches Vorgehen in der Fertigung kann wirtschaftlich erhebliche Vorteile bringen, da große plattenförmige Rohlinge bedampft werden können, aus denen erst später einzelne Düsenplatten erstellt werden. Bei ausreichend großem Düsendurchmesser ist die Gefahr der Verkeimung innerhalb der Düsen gering, zumindest dann, wenn innenseitig und/oder außenseitig die flächigen Bereiche der Düsenplatte mit einer entsprechenden Schicht versehen sind.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der stark schematisierten Figuren erläutert sind.
Die Figuren 1a bis 1c zeigen verschiedene Ausgestaltungen von Düsenplatten für erfindungsgemäße Inhalationseinrichtungen.
Fig. 2 sowie 3a und 3b zeigen besonders einfache Ausgestaltungen von Inhalationseinrichtungen, die nicht Teil der Erfindung sind.
Fig. 4a und 4b sowie 5a und 5b zeigen eine Inhalationseinrichtung mit einem
   Drehauslassventil gemäß die Erfindung.
Fig. 6a und 6b sowie Fig. 6c zeigen eine Inhalationseinrichtung mit einem Schwenk-Auslassventil, die nicht Teil der Erfindung ist.
Fig. 7a und 7b zeigen zusammen ein Inalationseinrichtungs-Set, das nicht Teil der Erfindung ist.
Fig. 8a und 8b zeigen eine weitere erfindungsgemäße Inhalationseinrichtung 10, die sich durch einen sehr einfachen Aufbau auszeichnet.
Fig. 9a und 9b zeigen eine Ergänzung der Inhalationseinrichtung der Fig. 8a und 8b in Form eines ankoppelbaren Mundstücks sowie einer ankoppelbaren Inhalationseinrichtung.

### DETAILLIERTE BESCHREIBUNG DER AUSFÜHRUNGSBEISPIELE

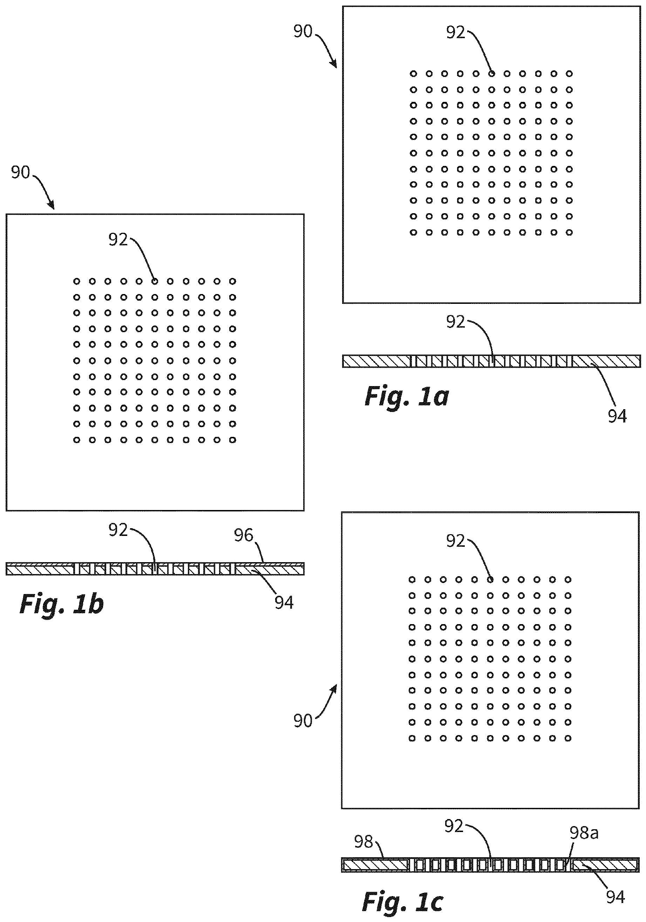

Die Fig. 1a bis 1c zeigen drei Düsenplatten 90 mit jeweils 121 Düsenöffnungen 92. Die Düsenöffnungen weisen einen Durchmesser von etwa 25 µm auf. Die dargestellten Düsenplatten 90 dienen der Verwendung in einer erfindungsgemäßen Inhalationseinrichtung. Dies wird nachfolgend noch beschrieben.

Die Düsenplatte 90 der Fig. 1a ist eine besonders einfache Düsenplatte, die als einheitlichen Werkstoff Silizium aufweist. In dieses Silizium sind die Düsenöffnungen 92 in einem Matrixraster eingebracht. Alternativ könnte zum Zwecke einer antibakteriellen Wirkung die Düsenplatte 90 der Fig. 1a auch als Ganzes aus Silber oder beispielsweise einer Silberlegierung oder einem anderen antibakteriell wirkenden Material gefertigt sein.

Die Fig. 1b zeigt eine Gestaltung, bei der die Düsenplatte 90 eine Grundplatte 94 und eine Beschichtung 96 aufweist. Während die Grundplatte 94 beispielsweise wiederum aus Silizium gefertigt sein kann, ist die Beschichtung 96 aus Silber oder einer Silberlegierung gebildet. Hierdurch wird Bakterienwachstum wirksam vermieden. Während bei Fig. 1b diese Beschichtung 96 nur einseitig dargestellt ist, kann dennoch auch eine zweiseitig beschichtete Platte vorgesehen sein. Eine Besonderheit der Gestaltung gemäß Fig. 1b ist, dass die Düsenöffnungen 92 nach der Beschichtung eingebracht wurden, so dass die Düsenöffnungen 92 innenseitig nicht oder nur partiell von der Beschichtung überdeckt sind. Dies kann aus Gründen einfacherer Herstellung einen Vorteil darstellen.

Die Gestaltung der Fig. 1c unterscheidet sich diesbezüglich. Auch hier ist eine Grundplatte 94 vorgesehen, die von einer Beschichtung 98 überdeckt wird. Da diese Beschichtung 98 jedoch nach Herstellen der Düsenöffnungen 92 aufgebracht wurde, überdeckt sie mit Abschnitten 98a auch die Innenseite der Düsenöffnungen 92.

Der partielle oder vollständige Überzug aus Silber oder einer Silberlegierung wirkt antibakteriell. Ist die Düsenplatte 90 auf der stromabwärtigen Seite beschichtet, so wirkt dies insbesondere einem Bakterienwachstum entgegen, das in dem niedergeschlagenen Flüssigkeitsfilm auf dieser Seite entstehen kann. Eine entsprechende Beschichtung auf der stromaufwärtigen Seite wirkt insbesondere auf die Flüssigkeit, die gegen Ende eines Austragvorgangs noch vor den Düsenöffnungen 92 steht. Dort ist eine antibakterielle Wirkung insbesondere deshalb zweckmäßig, da eine Verdunstung der dortigen Flüssigkeit durch die Düsenöffnungen 92 nur sehr langsam vonstattengeht und die Gefahr des Bakterienwachstums daher dort entsprechend groß ist.

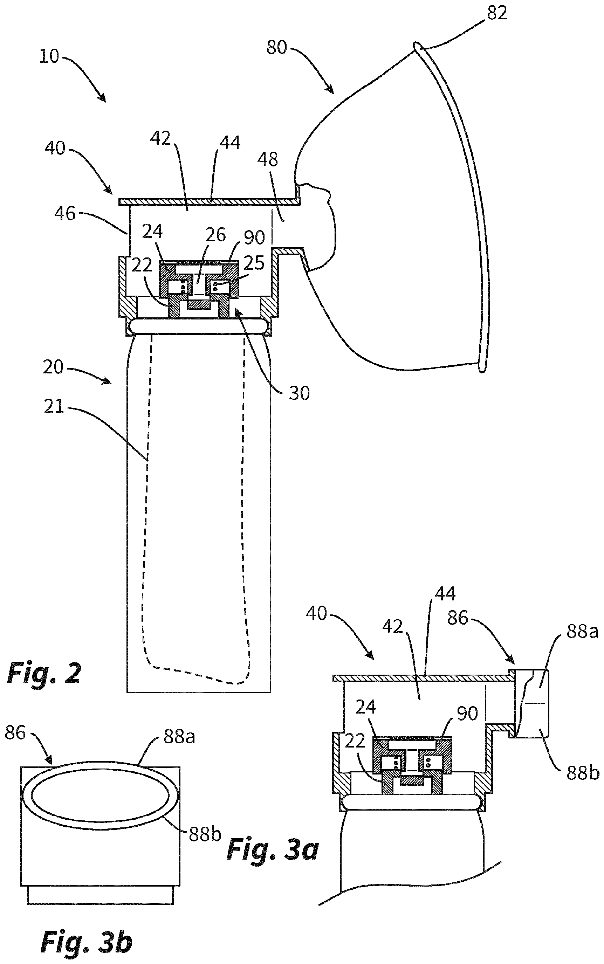

Die Fig. 2 sowie 3a und 3b zeigen sehr einfache Inhalationseinrichtungen 10 unter Verwendung solcher Düsenplatten 90. Die in den weiteren Figuren dargestellten Inhalationseinrichtungen bauen auf der grundsätzlichen Funktionalität dieser einfachen Inhalationseinrichtungen auf. Die Inhalationseinrichtung 10 der Fig. 2 verfügt über ein als Druckbehälter ausgestaltetes zylindrisches Gehäuse 20, in dem eine Salzlösung gelagert ist.

Alternativ könnte hier auch eine wässrige Lösung in Form einer Ringerlösung oder einer gepufferten Lösung, eine wässrige Lösung mit mindestens einem der Zusätze Kohlenhydrate, ätherische Öle, Menthol und Pflanzenextrakte, eine wässrige Lösung, enthaltend Vitamine, Spurenelemente, Mangan oder Zink, oder einer wässrige Lösung mit mindestens einem der Zusätze aus der Gruppe umfassend Zimtöl, Teebaumöl, Salbeiöl, Thymianöl, Zitronenmelissenöl gelagert sein.

Die Salzlösung kann mittels Treibgas druckbeaufschlagt sein. Eine Alternative hierzu liegt darin, dass der Druckbehälter mit Luft unter Überdruck befüllt ist und einen beutelförmigen Flüssigkeitsspeicher 21 aufweist, der in Fig. 2 exemplarisch gestrichelt dargestellt ist. Der Überdruck versucht, die Flüssigkeit aus dem Flüssigkeitsspeicher 21 in Richtung eines Austragkanals 26 und somit zur Düsenplatte 90 zu fördern. Zwischen der Düsenplatte 90 und dem Flüssigkeitsspeicher 21 ist ein Ventil 30 vorgesehen, welches gemeinsam durch einen zum Gehäuse 20 ortsfesten Stutzen 22 und einen demgegenüber in Hochrichtung verschieblichen Schieber 24 gebildet wird. Wie sich aus der Darstellung der Fig. 2 ergibt, muss der Schieber gegen die Kraft einer nicht dargestellten Feder niedergedrückt werden, damit der Austragkanal 26 geöffnet wird. Sobald dies der Fall ist, kann Flüssigkeit durch den Austragkanal 26 bis zur Düsenplatte 90 strömen. Unter dem sich dort dann einstellenden weitgehend konstanten Druck wird diese Flüssigkeit durch die Düsenöffnungen 92 der Düsenplatte 90 gedrückt und erzeugt so einen Inhalationsnebel.

Auf das Gehäuse 20 ist ein Applikatorkopf 40 aufgesetzt. Dieser umgibt einen Zerstäubungsraum 42, in den die durch die Düsenplatte 90 hindurch getretene Flüssigkeit in Nebelform gelangt. Das Gehäuse 44 des Applikatorkopfes 40 weist einen Lufteinlass 46 auf. Gegenüberliegend hierzu ist ein Luftauslass 48 vorgesehen, an den sich ein Applikationsstück anschließt. Das Applikationsstück in Fig. 2 ist eine Inhalationsmaske 80. Diese wird bestimmungsgemäß so am Gesicht des Benutzers angelegt, dass sie den Mund und die Nase vollständig überdeckt. Jeglicher Atemvorgang führt daher dazu, dass Luft durch den Applikatorkopf 40 hindurch angesogen wird, wo diese mittels der beschriebenen Vernebelung zur Inhalation vorbereitet wird.

Die Düsenplatte 90 kann wie auch bei den folgenden Ausführungsbeispielen in Art der Gestaltungen der Fig. 1a bis 1c ausgebildet sein. Eine nach außen weisende antibakterielle Schicht kann Bakterienwachstum verhindern, welches sich im Niederschlag des Inhalationsnebels auf der Düsenplatte 90 bilden kann. Eine zum Kanal 26 hin weisende Beschichtung sorgt dafür, dass in der Flüssigkeitsmenge, die innenseitig an der Düsenplatte 90 anliegt, kein Bakterienwachstum möglich ist. Dies ist besonders hilfreich, da die jenseits des Ventils 30 befindliche Flüssigkeit durch die Düsenöffnungen nur schwer verdunsten kann, so dass hier die Gefahr der Kontamination vergleichsweise hoch ist.

Die Fig. 3a und 3b zeigen die neben der Inhalationsmaske zweite Möglichkeit eines erfindungsgemäß zu nutzenden Applikatorstücks. Hierbei handelt es sich um ein Mundstück 86, welches entsprechend der Formgebung des menschlichen Mundes eine längliche Formgebung aufweist. Das Mundstück 86 ist an der Oberseite und der Unterseite mit Anlageflächen 88a, 88b versehen, die dem Anlegen der Lippen dienen und im Zusammenhang mit einigen der nachfolgend beschriebenen Ventileinrichtungen zweckmäßig sind, um hier ein Stützmoment einzukoppeln.

Bei den Ausgestaltungen der Fig. 2 sowie 3a und 3b ist die Art der Betätigung des Ventils nicht näher dargestellt. Das Niederdrücken des Schiebers könnte beispielsweise erfolgen, wenn dieser über einen nicht dargestellten, durch einen Schlitz in der Wandung des Gehäuses 44 geführten Betätigungshebel verfügt.

Besondere Ausgestaltungen des Ventils 30 bzw. der zugehörigen Öffnungsmechanik ergeben sich aus den folgenden Darstellungen.

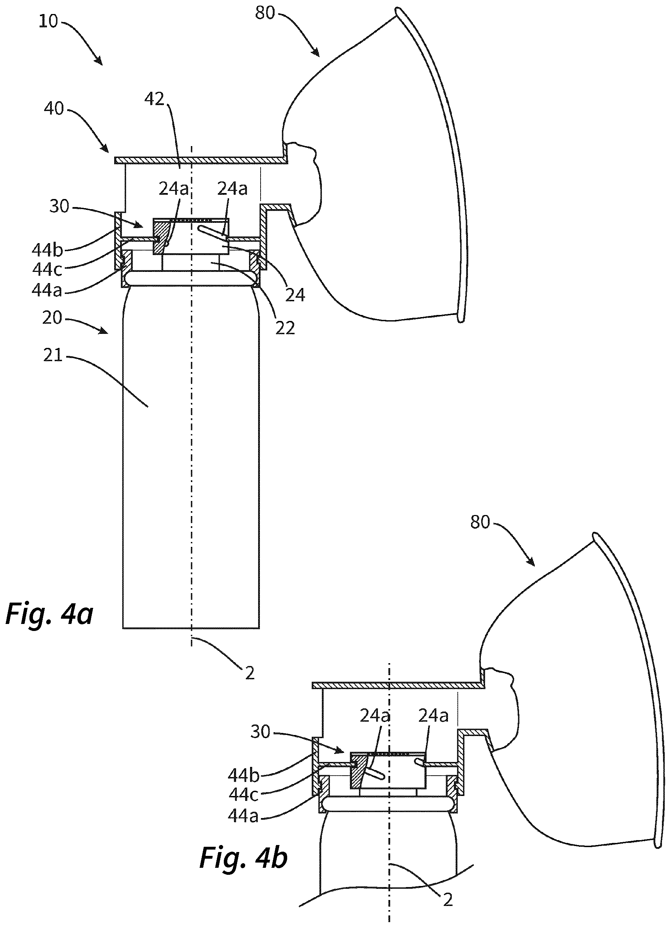

Bei der Ausgestaltung gemäß den Fig. 4a und 4b ist das Gehäuse des Applikatorkopfes 40 zweiteilig ausgebildet. Es weist einen unteren Abschnitt 44a auf, der verdrehgesichert am Gehäuse 20 angebracht ist. Demgegenüber ist um die Achse 2 ein oberer Abschnitt 44b drehbeweglich. Von diesem oberen Abschnitt 44b erstrecken sich zwei Ausleger 44c bis zum Schieber 24 und in am Schieber 24 vorgesehene schräggestellte Kulissennuten 24a. Da der Schieber 24 in nicht näher dargestellter Art und Weise drehfest zum Gehäuse 20 ausgestaltet ist, führt eine Drehung des oberen Gehäuseabschnitts 44b zusammen mit dem Applikationsstück 80 gegenüber dem Gehäuse 20 dazu, dass der Schieber 24 axial gegenüber dem Gehäuse 20 verschoben wird und somit ein Aktivieren und Deaktivieren der Inhalationsnebelerzeugung durch Öffnen und Schließen des Ventils 30 möglich ist. Von besonderem Vorteil ist, dass dieser Schaltvorgang einhändig erfolgen kann. Während der Benutzung der Inhalationseinrichtung 10 wird üblicherweise das Gehäuse 20 mit einer Hand umgriffen und hierdurch auch die Inhalationsmaske 80 gegen das Gesicht gedrückt. In dieser Stellung kann das Gehäuse 20 einfach als Ganzes und gemeinsam mit dem Schieber 24 gedreht werden, um die Erzeugung des Inhalationsnebels zu initiieren.

Diese einhändige Bedienung wird dadurch begünstigt, dass das Applikatorstück durch seine gegenüber der Achse 2 exzentrische Anordnung so ausgestaltet ist, dass es bei einer Dreh- oder Schwenkbewegung des Gehäuses 20 gegenüber dem Applikatorstück die Einkopplung eines Stützmomentes gestattet. Im Falle der Inhalationsmaske 80 wird dies durch eine umlaufende Dichtkante 82 erreicht, die am Gesicht anliegt und sich dort abstützen kann. Im Falle des Mundstücks 86 wird dies durch die für die Anlage der Lippen vorgesehenen Anlagefläche 88a, 88b erreicht. Durch leichtes Zusammendrücken der Lippen wird das Mundstück so fixiert, dass eine Relativbewegung des Gehäuses 20 zum Zwecke des Schaltens eines Ventils leicht möglich ist.

Fig. 4a zeigt die Inhalationseinrichtung 10 bei geschlossenem Austragventil 30. Die Fig. 4b zeigt den offenen Zustand.

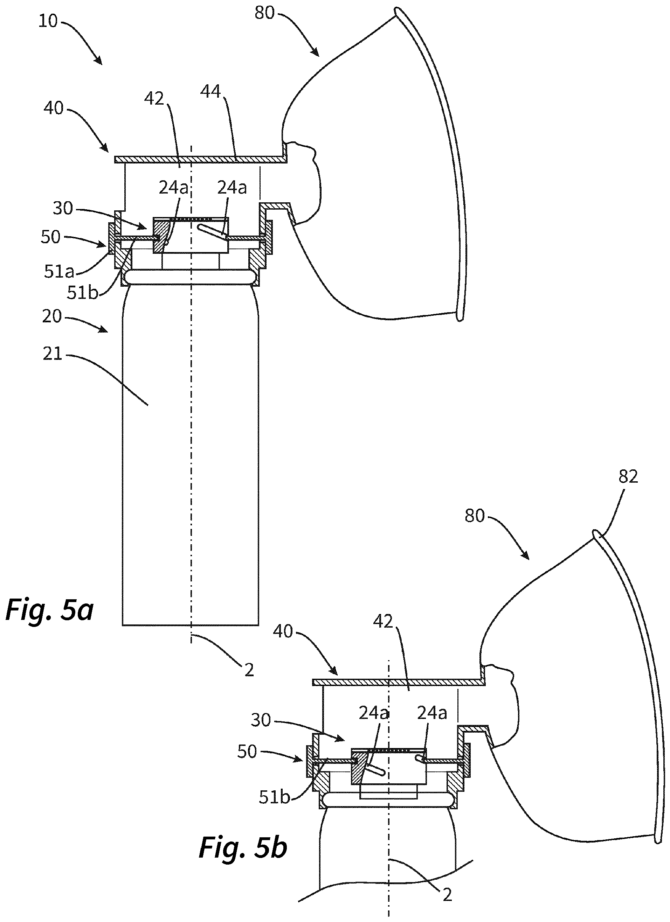

Die Gestaltung der Fig. 5a und 5b ist eine Variante zur Gestaltung der Fig. 4a und 4b. Das Gehäuse 44 des Applikatorkopfes 40 ist hierbei wiederum einstückig vorgesehen. Allerdings ist ein separates Schaltelement 50 am Applikatorkopf 40 angebracht, welches ebenfalls um die Drehachse 2 drehbar ist. Das Schaltelement 50 verfügt über einen von außen zugänglichen Schaltring 51a und nach innen in die Kulissenspuren des Schiebers 24 ragende Ausleger 51b. Durch Drehen des Schaltelements 50 über den Schaltring 51a kann das Ventil 30 geöffnet und geschlossen werden.

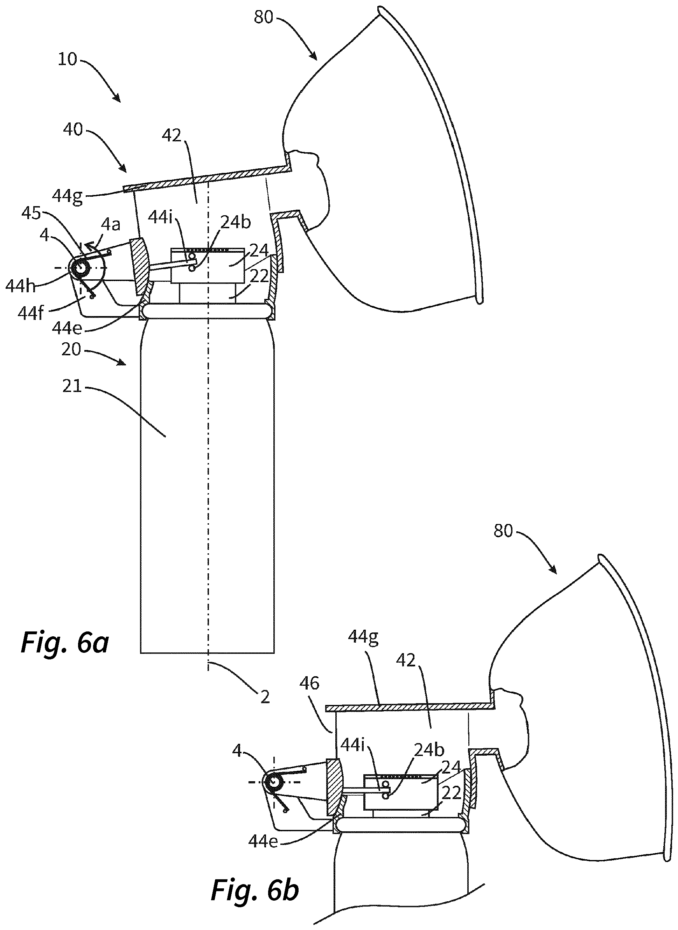

Bei der Gestaltung gemäß den Fig. 6a und 6b weist das Gehäuse 44 des Austragkopfes 40 wiederum eine Zweiteilung auf. Ein unterer Gehäuseabschnitt 44e ist fest am Gehäuse 20 angebracht. Ein oberer Gehäuseabschnitt 44g, an dem auch die Inhalationsmaske 80 vorgesehen ist, ist demgegenüber um eine Schwenkachse 4 schwenkbar. Zu diesem Zweck verfügen sowohl der untere Gehäuseabschnitt 44e als auch der obere Gehäuseabschnitt 44g über aneinander schwenkbar angelenkte Ausleger 44f, 44h, die gelenkig aneinander angebracht sind. Zwischen diesen Auslegern ist eine Schenkelfeder 45 vorgesehen, die den oberen Gehäuseabschnitt 44g gegenüber dem unteren Gehäuseabschnitt 44e in Richtung des Pfeils 4a momentenbeaufschlagt.

Zur Betätigung des Ventils 30 ist ein Ausleger 44i am oberen Gehäuseabschnitt 44g vorgesehen, der mit einem Nocken 24b am Schieber 24 zusammenwirkt. Wenn eine nur geringfügige Schwenkbewegung um einige Grad gegenüber der Ausgangsposition der Fig. 6a erfolgt, drückt der Ausleger 44b den Schieber 24 gegenüber dem Gehäuse 20 durch Kraftbeaufschlagung des Nocken 24b nach unten und bewirkt so das Öffnen des Ventils 30.

Wie auch bei den Ausgestaltungen der Fig. 4a und 4b ist hier eine sehr einfache einhändige Handhabung möglich. Wenn die Inhalationsmaske 80 gegen das Gesicht gedrückt wird, kann die Schwenkbewegung des Gehäuses 20 und damit das Öffnen und Schließen des Ventils 30 einhändig bewirkt werden.

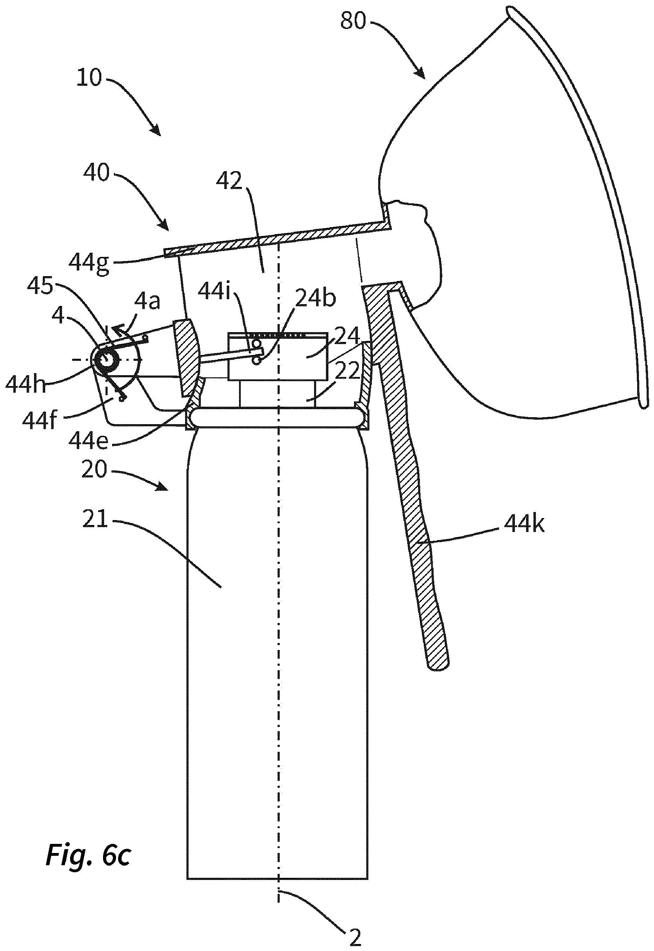

Fig. 6c zeigt eine ergänzte Gestaltung bei der zusätzlich zu den Komponenten der Ausführungsform der Fig. 6a und 6b ein Betätigungshebel 44k am Gehäuse des Applikatorkopfes 40 vorgesehen ist, Durch Umgreifen des Gehäuses 20 und des Betätigungshebels 44k mit einer Hand und Zusammenziehen der Hand kann ein Verschwenken und damit ein Öffnen des Ventils verursacht werden.

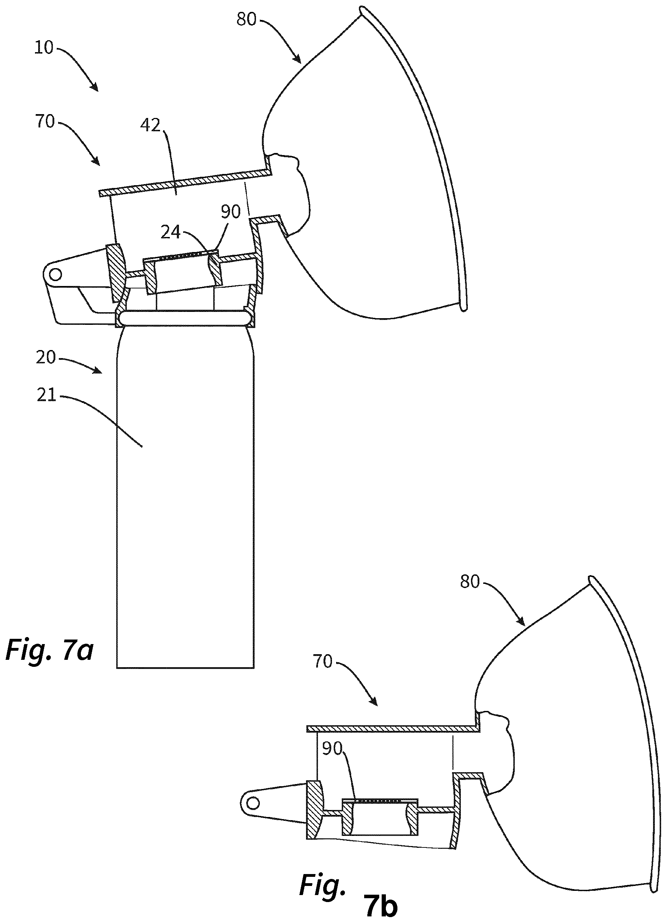

Fig. 7a zeigt eine Inhalationseinrichtung 10, die derjenigen der Fig. 6a und 6b ähnelt. Die Besonderheit ist hier, dass der Schieber 24 in den oberen Gehäuseabschnitt integriert ist. Das Ventil sollte dabei so beschaffen sein, dass es bei Abnahme des Schiebers 24 schließt.

Es ergibt sich eine zusammenhängende Baueinheit 70, die separat in Fig. 7b dargestellt ist und die ein einfaches Reinigen gestattet.

Ein erfindungsgemäßes Inhalationseinrichtungsset umfasst mehrere dieser Baueinheiten 70, so dass diese aus hygienischen Gründen getauscht werden können.

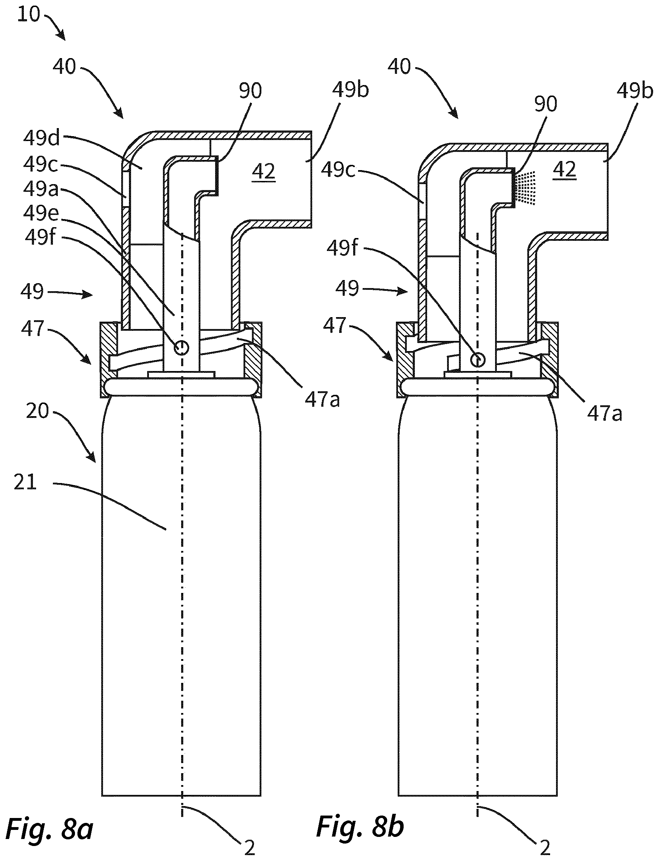

Fig. 8a und 8b zeigen eine weitere Inhalationseinrichtung 10. Bei dieser ist ein Applikatorkopf 40 aus sehr wenigen Bauteilen aufgebaut und daher günstig in der Herstellung.

Der Applikatorkopf verfügt über eine Basis 47, die drehfest mit dem Gehäuse 20 verbunden ist. Die Basis weist die Form eines Rings auf, wobei an der Innenseite einander gegenüberliegend zwei wendelabschnittsförmige Kulissenspuren 47a vorgesehen sind.

In die Basis 47 eingesetzt ist ein Auslassteil 49. Dieses verfügt über ein gekrümmtes Außenrohr 49a, welches in einer als Mundstück verwendbaren Öffnung 49b endet. Das Außenrohr 49a verfügt über eine Einströmöffnung 49c auf der der Öffnung 49b gegenüberliegenden Seite.

Im Außenrohr 49a ortsfest und vorzugsweise einstückig mit dem Außenrohr 49a verbunden ist ein Innenrohr 49e, welches vorliegend über eine Rippe 49d mit dem Außenrohr 49a verbunden ist. Das Innenrohr endet in einer Düsenplatte 90. Flüssigkeit, die durch das Innenrohr zur Düsenplatte 90 gefördert wird, wird dort in die Zerstäubungskammer 42 ausgetragen. Der Benutzer kann die zerstäubte Flüssigkeit durch Saugen an der Öffnung 49b inhalieren.

Zum Steuern der Zerstäubung ist das Auslassteil 49 gegenüber der Basis 47 um die Achse 2 drehbar. Am Auslassteil sind Führungsbolzen 49f vorgesehen, die sich vorliegend von einer Außenseite des Innenrohrs 49e nach außen bis in die Kulissenspuren 47a erstrecken. Eine Drehbewegung des Auslassteils 49 gegenüber der Basis 47 bewirkt daher auch eine axiale Relativverlagerung in Richtung der Achse 2. Dies verursacht das Öffnen und Schließen eines Ventils, welches in nicht dargestellter Weise innerhalb des Gehäuses 20 vorgesehen ist.

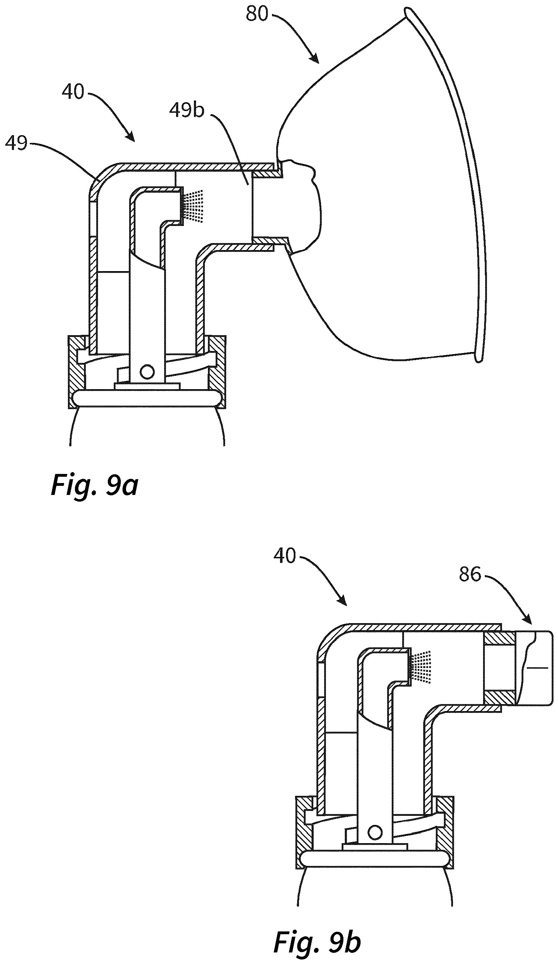

Das Ende des Außenrohrs 49 mit der Öffnung 49b kann unmittelbar als Mundstück verwendet werden. Die Fig. 9a und 9b verdeutlichen jedoch eine weitere Möglichkeit. So kann einer Inhalationseinrichtung mit einem Applikatorkopf entsprechend der Fig. 8a und 8b auch ein separates Mundstück 86 oder eine Inhalationsmaske 80 beigelegt sein. Diese umfassen jeweils einen Steckabschnitt, der in die Öffnung 49b eingeschoben werden kann und dort formschlüssig oder kraftschlüssig gehalten wird. So kann die Inhalationseinrichtung an individuellen Bedarf angepasst werden.

## Patentansprüche

1. Inhalationseinrichtung (10) zum Inhalieren einer Flüssigkeit in vernebelter Form mit den folgenden Merkmalen:
a. die Inhalationseinrichtung (10) verfügt über ein Gehäuse (20), das zumindest abschnittsweise als rotationssymmetrischer Körper ausgestaltet ist, dessen Mittelachse eine Haupterstreckungsachse (2) des Gehäuses (20) definiert, und welches einen Flüssigkeitsspeicher (21) umgibt, in dem die Flüssigkeit vor dem Austrag durch Beaufschlagung mit Treibgas, mit Druckluft oder durch eine vorgespannte Federeinrichtung unter Druck stehend gelagert ist, und
b. die Inhalationseinrichtung (10) verfügt über einen Applikatorkopf (40) mit
- einem Zerstäubungsraum (42), und
- einem mit dem Zerstäubungsraum (42) verbundenen Applikatorstück (80; 86), das seitlich am Applikatorkopf vorgesehen ist, wobei das Applikatorstück (80; 86) entweder als Mundstück (86) zur Aufnahme im Mund eines Patienten oder als Inhalationsmaske (80) zur dichten Überdeckung des Mundes, der Nase oder des Mundes und der Nase oder als Adapterstück zur Anbringung eines Mundstücks (86) oder einer Inhalationsmaske (80) ausgebildet ist,
c. die Inhalationseinrichtung (10) verfügt über einen Austragkanal (26), der den Flüssigkeitsspeicher (21) mit dem Applikatorkopf (40) verbindet,
d. die Inhalationseinrichtung (10) verfügt über ein schaltbares Dreh-Auslassventil (30), durch das der Austragkanal (26) geöffnet und geschlossen werden kann, **gekennzeichnet dadurch, dass**
e. das Dreh-Auslassventil (30) ist durch eine Drehbewegung des Gehäuses (20) um die Haupterstreckungsachse (2) gegenüber dem Applikatorstück (80; 86) schaltbar.

2. Inhalationseinrichtung (10) nach Anspruch 1 mit dem folgenden Merkmal:
a. es ist ein Lufteinlass (46) vorgesehen, der in den Zerstäubungsraum (42) mündet.

3. Inhalationseinrichtung (10) nach Anspruch 1 oder 2 mit dem folgenden Merkmal:
a. die Inhalationseinrichtung (10) verfügt am Ende des Austragkanals (26) über eine Düsenplatte (90) mit einer Vielzahl von Düsenöffnungen (92) zur Erzeugung eines Inhalationsnebels, durch die hindurch die Flüssigkeit vom Flüssigkeitsspeicher (21) in den Zerstäubungsraum (42) geleitet wird.

4. Inhalationseinrichtung nach einem der vorstehenden Ansprüche mit dem Merkmal:
a. eine durch das Applikatorstück (80; 86) definierte Austragrichtung ist gegenüber der Haupterstreckungsachse (2) des Gehäuses (20) angewinkelt, wobei vorzugsweise die Austragrichtung und die Haupterstreckungsachse (2) einen Winkel zwischen 10° und 170° einschließen, insbesondere vorzugsweise einen Winkel zwischen 60° und 120°.

5. Inhalationseinrichtung nach Anspruch 2 mit dem Merkmal:
a. der Lufteinlass (46) und das Applikatorstück (80; 86) sind auf gegenüberliegenden Seiten des Zerstäubungsraumes (42) vorgesehen.

6. Inhalationseinrichtung (10) nach einem der vorstehenden Ansprüche mit dem Merkmal:
a. dem Auslassventil (30) ist eine Federeinrichtung (25, 45, 55) zugeordnet, die derart ausgebildet ist, dass ihre Federkraft stets versucht, das Auslassventil (30) zu schließen.

7. Inhalationseinrichtung (10) nach einem der vorstehenden Ansprüche mit den Merkmalen:
a. der Applikatorkopf (40) verfügt über ein drehfest mit dem Gehäuse verbundenes Basisteil (47) und über demgegenüber drehbares Auslassteil (49), welches den Zerstäubungsraum (42) umfasst, und
b. das Basisteil (47) und das Auslassteil (49) sind aneinander derart geführt, dass eine Drehbewegung des Auslassteils (49) gegenüber dem Basisteil (47) eine axiale Verlagerung der Auslassteils (49) gegenüber dem Basisteil (47) bewirkt, durch die ein Auslassventil der Inhalationseinrichtung (10) schaltbar ist.

8. Inhalationseinrichtung (10) nach Anspruch 3 oder Ansprüche 4 bis 7 wenn abhängig von Anspruch 3 mit mindestens einem der folgenden Merkmale:
a. der Austragkanal (26) ist zumindest abschnittsweise mit einem antibakteriell wirkenden Material versehen, insbesondere mit Silber oder einer Silberlegierung, oder
b. das Mundstück (86) weist einen Auslass auf, dessen Breite größer ist als seine Höhe und der an der Oberseite und an der Unterseite über Anlageflächen (88a, 88b) zum Anlegen der Oberlippe und der Unterlippe verfügt, oder
c. die Inhalationsmaske (80) weist eine umlaufende Dichtkante (82) zur Anlage am Gesicht eines Benutzers auf, wobei die Dichtkante (82) so bemessen ist, dass sie den Mund oder die Nase oder den Mund und die Nase umschließen kann, oder
d. die Düsenplatte (90) umfasst Düsenöffnungen (92) mit einem Durchmesser zwischen 1 µm und 100 µm, insbesondere zwischen 2 µm und 10 µm, oder
e. die Düsenplatte (90) umfasst mindestens 9 Düsenöffnungen (92), vorzugsweise mindestens 16 Düsenöffnungen (92) und insbesondere vorzugsweise mindestens 25 Düsenöffnungen (92), oder
f. das Applikatorstück ist als Adapterstück zur Anbringung eines Mundstücks (80) oder einer Inhalationsmaske (86) ausgebildet und die Inhalationseinrichtung (10) verfügt weiterhin über ein Mundstück (86) zur Aufnahme im Mund eines Patienten oder eine Inhalationsmaske (80) zur dichten Überdeckung des Mundes, der Nase oder des Mundes und der Nase, oder
g. die Inhalationseinrichtung (10) verfügt über eine Betätigungshandhabe (44k), die gegenüber dem Gehäuse (20) verlagerbar, vorzugsweise verschwenkbar, ist, um hierdurch ein Auslassventil der Inhalationseinrichtung (10) zu öffnen.

9. Inhalationseinrichtung (10) nach Anspruch 3 oder Ansprüche 4 bis 8 wenn abhängig von Anspruch 3 mit dem Merkmal:
a. das Applikatorstück (80; 86) und die Düsenplatte (90) sind Teil einer gemeinsamen Baueinheit (70), die werkzeuglos vom Gehäuse (20) lösbar ist.

10. Inhalationseinrichtung (10) nach Anspruch 3 oder Ansprüche 4 bis 9 wenn abhängig von Anspruch 3 mit mindestens einem der folgenden Merkmale:
a. die Düsenplatte (90) ist zumindest abschnittsweise aus Silber oder aus einem Silber beinhaltenden Material gebildet, oder
b. die Düsenplatte (90) umfasst Düsenöffnungen (92) mit einem Durchmesser zwischen 1 µm und 100 µm, insbesondere zwischen 2 µm und 10 µm, oder
c. die Düsenplatte (90) umfasst mindestens 9 Düsenöffnungen (92), vorzugsweise mindestens 16 Düsenöffnungen (92) und insbesondere vorzugsweise mindestens 25 Düsenöffnungen (92).

11. Inhalationseinrichtung (10) nach Anspruch 3 oder Ansprüche 4 bis 10 wenn abhängig von Anspruch 3 mit den folgenden Merkmalen:
a. die Düsenplatte (90) weist eine Grundplatte (94) aus einem Material auf, welches kein Silber oder Silber beinhaltendes Material beinhaltet,
b. auf der Grundplatte (90) ist eine Schicht (96; 98) aus Silber oder aus einem Silber beinhaltenden Material vorgesehen,
vorzugsweise mit einem der folgenden weiteren Merkmale:
c. die Schicht (96; 98) aus Silber oder Silber oder aus einem Silber beinhaltenden Material ist nach Einbringung der Düsenöffnungen (92) durch Bedampfen oder Eintauchen auf die Grundplatte (94) aufgebracht, so dass die Düsenöffnungen (92) an ihren Innenseiten zumindest teilweise mit dieser Schicht bedeckt sind, oder
d. die Düsenöffnungen (92) sind nach Bedampfen oder Eintauchen der Grundplatte (94) zum Zwecke der Herstellung der Schicht aus Silber oder Silberoxid eingebracht, so dass die Düsenöffnungen (92) an ihrer Innenseite zumindest teilweise frei von dieser Schicht sind.

12. Inhalationseinrichtung (10) nach einem der Ansprüche 3 bis 11 mit dem folgenden Merkmal:
a. der Flüssigkeitsspeicher ist mit einer Flüssigkeit befüllt, die ausgewählt ist aus der Gruppe umfassend die folgenden Flüssigkeiten:
- einer salzhaltigen wässrigen Lösung,
- einer wässrigen Lösung in Form einer Ringerlösung oder einer gepufferten Lösung,
- einer wässrigen Lösung mit mindestens einem der Zusätze Kohlenhydrate, ätherische Öle, Menthol und Pflanzenextrakte,
- einer wässrigen Lösung, enthaltend Vitamine, Spurenelemente, Mangan oder Zink, oder
- einer wässrigen Lösung mit mindestens einem der Zusätze aus der Gruppe umfassend Zimtöl, Teebaumöl, Salbeiöl, Thymianöl, Zitronenmelissenöl.

13. Inhalationseinrichtungs-Set mit den Merkmalen:
a. das Set umfasst eine Inhalationseinrichtung (10) nach einem der vorstehenden Ansprüche 3 bis 12, und
b. das Set umfasst mindestens zwei Baueinheiten (70), die jeweils ein Applikatorstück (80; 86) und eine Düsenplatte (90) umfassen.

## Claims

1. Inhalation device (10) for inhaling a liquid in nebulized form, with the following features:
a. the inhalation device (10) has a housing (20) which is configured at least in part as a rotationally symmetrical body, of which the centre axis defines a main axis of extent (2) of the housing (20), and which encloses a liquid reservoir (21) in which the liquid is stored in pressurized form before being discharged when acted upon by propellant gas, by compressed air or by a pretensioned spring mechanism, and
b. the inhalation device (10) has an applicator head (40) with
- a nebulization chamber (42), and
- an applicator piece (80; 86) which is connected to the nebulization chamber (42) and provided laterally on the applicator head, wherein the applicator piece (80; 86) is designed either as a mouthpiece (86), to be received in the mouth of a patient, or as an inhalation mask (80), to sealingly cover the mouth, the nose, or the mouth and the nose, or as an adapter piece for fitting a mouthpiece (86) or an inhalation mask (80),
c. the inhalation device (10) has a discharge channel (26) which connects the liquid reservoir (21) to the applicator head (40),
d. the inhalation device (10) has a switchable rotary outlet valve (30), by which the discharge channel (26) can be opened and closed, **characterized in that**
e. the rotary outlet valve (30) is switchable with respect to the applicator piece (80; 86) by a rotation movement of the housing (20) about the main axis of extent (2).

2. Inhalation device (10) according to Claim 1, with the following feature:
a. an air inlet (46) is provided which opens into the nebulization chamber (42).

3. Inhalation device (10) according to Claim 1 or 2, with the following feature:
a. the inhalation device (10) has, at the end of the discharge channel (26), a nozzle plate (90) with a large number of nozzle openings (92) which serve to generate an inhalation mist and through which the liquid from the liquid reservoir (21) is conveyed into the nebulization chamber (42).

4. Inhalation device according to one of the preceding claims, with the feature:
a. a discharge direction defined by the applicator piece (80; 86) is angled with respect to the main axis of extent (2) of the housing (20), wherein the discharge direction and the main axis of extent (2) preferably enclose an angle of between 10° and 170°, particularly preferably an angle of between 60° and 120°.

5. Inhalation device according to Claim 2, with the feature:
a. the air inlet (46) and the applicator piece (80; 86) are provided on opposite sides of the nebulization chamber (42).

6. Inhalation device (10) according to one of the preceding claims, with the feature:
a. the outlet valve (30) is assigned a spring mechanism (25, 45, 55), which is configured in such a way that its spring force always seeks to close the outlet valve (30).

7. Inhalation device (10) according to one of the preceding claims, with the features:
a. the applicator head (40) has a base part (47), which is connected in a rotationally fixed manner to the housing, and an outlet part (49), which is rotatable relative to the base part (47) and which comprises the nebulization chamber (42), and
b. the base part (47) and the outlet part (49) are guided on each other in such a way that a rotation movement of the outlet part (49) relative to the base part (47) causes an axial displacement of the outlet part (49) relative to the base part (47), by means of which axial displacement an outlet valve of the inhalation device (10) is switchable.

8. Inhalation device (10) according to Claim 3 or Claims 4 to 7 if dependent on Claim 3, with at least one of the following features:
a. the discharge channel (26) is provided at least in part with a material having an antibacterial action, in particular with silver or a silver alloy, or
b. the mouthpiece (86) has an outlet which has a width greater than its height and which, on the top face and on the bottom face, has bearing surfaces (88a, 88b) for bearing on the upper lip and the lower lip, or
c. the inhalation mask (80) has a circumferential sealing edge (82) for bearing on the face of a user, wherein the sealing edge (82) is dimensioned such that it can enclose the mouth or the nose or the mouth and the nose, or
d. the nozzle plate (90) comprises nozzle openings (92) with a diameter of between 1 µm and 100 µm, in particular between 2 µm and 10 µm, or
e. the nozzle plate (90) comprises at least 9 nozzle openings (92), preferably at least 16 nozzle openings (92) and particularly preferably at least 25 nozzle openings (92), or
f. the applicator piece is designed as an adapter piece for fitting a mouthpiece (80) or an inhalation mask (86), and the inhalation device (10) moreover has a mouthpiece (86), to be received in the mouth of a patient, or an inhalation mask (80), to sealingly cover the mouth, the nose, or the mouth and the nose, or
g. the inhalation device (10) has an actuation handle (44k), which is movable, preferably pivotable, with respect to the housing (20) in order thereby to open an outlet valve of the inhalation device (10).

9. Inhalation device (10) according to Claim 3 or Claims 4 to 8 if dependent on Claim 3, with the feature:
a. the applicator piece (80; 86) and the nozzle plate (90) are part of a common structural unit (70), which is detachable from the housing (20) without the use of tools.

10. Inhalation device (10) according to Claim 3 or Claims 4 to 9 if dependent on Claim 3, with at least one of the following features:
a. the nozzle plate (90) is formed at least in part from silver or from a silver-containing material, or
b. the nozzle plate (90) comprises nozzle openings (92) with a diameter of between 1 µm and 100 µm, in particular between 2 µm and 10 µm, or
c. the nozzle plate (90) comprises at least 9 nozzle openings (92), preferably at least 16 nozzle openings (92) and particularly preferably at least 25 nozzle openings (92).

11. Inhalation device (10) according to Claim 3 or Claims 4 to 10 if dependent on Claim 3, with the following features:
a. the nozzle plate (90) has a main plate (94) made of a material that does not contain silver or silver-containing material,
b. a layer (96; 98) of silver or of a silver-containing material is provided on the main plate (90),
preferably with one of the following further features:
c. the layer (96; 98) of silver or of silver or of a silver-containing material is applied to the main plate (94) by vapour deposition or by immersion after the nozzle openings (92) have been formed, such that the nozzle openings (92) are at least partially covered with this layer on their insides, or
d. the nozzle openings (92) are formed after vapour deposition or immersion of the main plate (94) for the purpose of producing the layer of silver or silver oxide, such that the nozzle openings (92) are at least partially free of this layer on their inside.

12. Inhalation device (10) according to one of Claims 3 to 11, with the following feature:
a. the liquid reservoir is filled with a liquid chosen from the group comprising the following liquids:
- a saline aqueous solution,
- an aqueous solution in the form of a Ringer's solution or a buffered solution,
- an aqueous solution with at least one of the additives carbohydrates, essential oils, menthol and plant extracts,
- an aqueous solution containing vitamins, trace elements, manganese or zinc, or
- an aqueous solution with at least one of the additives from the group comprising cinnamon oil, tea tree oil, sage oil, thyme oil, lemon balm oil.

13. Inhalation device set with the features:
a. the set comprises an inhalation device (10) according to one of preceding Claims 3 to 12, and
b. the set comprises at least two structural units (70), which each comprise an applicator piece (80; 86) and a nozzle plate (90).

## Revendications

1. Dispositif d'inhalation (10) pour l'inhalation d'un liquide sous forme atomisée, comprenant les caractéristiques suivantes :
a. le dispositif d'inhalation (10) dispose d'un boîtier (20) qui est configuré au moins en partie sous forme de corps à symétrie de révolution, dont l'axe médian définit un axe d'étendue principale (2) du boîtier (20), et qui entoure un réservoir de liquide (21) dans lequel le liquide, avant son expulsion par sollicitation avec du gaz propulseur, est stocké sous pression avec de l'air comprimé ou par un dispositif de ressort précontraint, et
b. le dispositif d'inhalation (10) dispose d'une tête d'applicateur (40) comprenant
- un espace de vaporisation (42), et
- une pièce d'applicateur (80 ; 86) connectée à l'espace de vaporisation (42), qui est prévue latéralement sur la tête d'applicateur, la pièce d'applicateur (80 ; 86) étant réalisée sous forme d'embout (86) destiné à être reçu dans la bouche d'un patient ou sous forme de masque d'inhalation (80) destiné à couvrir hermétiquement la bouche, le nez, ou la bouche et le nez, ou sous forme de pièce d'adaptateur pour le montage d'un embout (86) ou d'un masque d'inhalation (80),
c. le dispositif d'inhalation (10) dispose d'un canal d'expulsion (26) qui relie le réservoir de liquide (21) à la tête d'applicateur (40),
d. le dispositif d'inhalation (10) dispose d'une soupape de sortie rotative commutable (30) par le biais de laquelle le canal d'expulsion (26) peut être ouvert et fermé,
**caractérisé en ce que**
e. la soupape de sortie rotative (30) peut être commutée par un mouvement de rotation du boîtier (20) autour de l'axe d'étendue principale (2) par rapport à la pièce d'applicateur (80 ; 86).

2. Dispositif d'inhalation (10) selon la revendication 1, comprenant la caractéristique suivante :
a. il est prévu une entrée d'air (46) qui débouche dans l'espace de vaporisation (42).

3. Dispositif d'inhalation (10) selon la revendication 1 ou 2, comprenant la caractéristique suivante :
a. le dispositif d'inhalation (10), à l'extrémité du canal d'expulsion (26), dispose d'une plaque à buses (90) avec une pluralité d'ouvertures de buses (92) pour générer un brouillard d'inhalation, à travers lequel le liquide est guidé depuis le réservoir de liquide (21) dans l'espace de vaporisation (42).

4. Dispositif d'inhalation selon l'une quelconque des revendications précédentes, comprenant la caractéristique suivante :
a. une direction d'expulsion définie par la pièce d'applicateur (80 ; 86) est inclinée par rapport à l'axe d'étendue principale (2) du boîtier (20), la direction d'expulsion et l'axe d'étendue principale (2) formant de préférence un angle compris entre 10° et 170°, en particulier de préférence un angle compris entre 60° et 120°.

5. Dispositif d'inhalation selon la revendication 2, comprenant la caractéristique suivante :
a. l'entrée d'air (46) et la pièce d'applicateur (80 ; 86) sont prévues sur des côtés opposés de l'espace de vaporisation (42).

6. Dispositif d'inhalation (10) selon l'une quelconque des revendications précédentes, comprenant la caractéristique suivante :
a. la soupape de sortie (30) est associée à un dispositif de ressort (25, 45, 55) qui est réalisé de telle sorte que sa force de ressort ait tendance à toujours fermer la soupape de sortie (30).

7. Dispositif d'inhalation (10) selon l'une quelconque des revendications précédentes, comprenant les caractéristiques suivantes :
a. la tête d'applicateur (40) dispose d'une partie de base (47) connectée de manière solidaire en rotation au boîtier et d'une partie de sortie (49) pouvant tourner par rapport à celle-ci, qui comprend l'espace de vaporisation (42), et
b. la partie de base (47) et la partie de sortie (49) sont guidées l'une contre l'autre de telle sorte qu'un mouvement de rotation de la partie de sortie (49) par rapport à la partie de base (47) provoque un décalage axial de la partie de sortie (49) par rapport à la partie de base (47), par le biais duquel une soupape de sortie du dispositif d'inhalation (10) peut être commutée.

8. Dispositif d'inhalation (10) selon la revendication 3 ou selon les revendications 4 à 7 lorsqu'elles dépendent de la revendication 3, comprenant au moins l'une des caractéristiques suivantes :
a. le canal d'expulsion (26) est pourvue au moins en partie d'un matériau à effet antibactérien, en particulier d'argent ou d'un alliage d'argent, ou
b. l'embout (86) présente une sortie dont la largeur est supérieure à sa hauteur et qui dispose, au niveau du côté supérieur et au niveau du côté inférieur, de surfaces d'application (88a, 88b) pour l'application de la lèvre supérieure et de la lèvre inférieure, ou
c. le masque d'inhalation (80) présente un bord d'étanchéité périphérique (82) pour l'application contre le visage d'un utilisateur, le bord d'étanchéité (82) étant dimensionné de manière à pouvoir entourer la bouche ou le nez ou la bouche et le nez, ou
d. la plaque à buses (90) comprend des ouvertures de buses (92) ayant un diamètre compris entre 1 µm et 100 µm, en particulier entre 2 µm et 10 µm, ou
e. la plaque à buses (90) comprend au moins 9 ouvertures de buses (92), de préférence au moins 16 ouvertures de buses (92) et notamment de préférence au moins 25 ouvertures de buses (92), ou
f. la pièce d'applicateur est réalisée sous forme de pièce d'adaptateur pour le montage d'un embout (80) ou d'un masque d'inhalation (86) et le dispositif d'inhalation (10) dispose en outre d'un embout (86) destiné à être reçu dans la bouche d'un patient ou un masque d'inhalation (80) pour recouvrir hermétiquement la bouche, le nez ou la bouche et le nez, ou
g. le dispositif d'inhalation (10) dispose d'une manette d'actionnement (44k) qui peut être décalée, de préférence pivotée, par rapport au boîtier (20) pour ouvrir ainsi une soupape de sortie du dispositif d'inhalation (10).

9. Dispositif d'inhalation (10) selon la revendication 3 ou selon les revendications 4 à 8 lorsqu'elles dépendent de la revendication 3, comprenant la caractéristique suivante :
a. la pièce d'applicateur (80 ; 86) et la plaque à buses (90) font partie d'une unité structurelle commune (70) qui peut être détachée du boîtier (20) sans outil.

10. Dispositif d'inhalation (10) selon la revendication 3 ou selon les revendications 4 à 9 lorsqu'elles dépendent de la revendication 3, comprenant au moins l'une des caractéristiques suivantes :
a. la plaque à buses (90) est formée au moins en partie d'argent ou d'un matériau contenant de l'argent, ou
b. la plaque à buses (90) comprend des ouvertures de buses (92) ayant un diamètre compris entre 1 µm et 100 µm, en particulier entre 2 µm et 10 µm, ou
c. la plaque à buses (90) comprend au moins 9 ouvertures de buses (92), de préférence au moins 16 ouvertures de buses (92) et en particulier de préférence au moins 25 ouvertures de buses (92).

11. Dispositif d'inhalation (10) selon la revendication 3 ou les revendications 4 à 10 lorsqu'elles dépendent de la revendication 3, comprenant les caractéristiques suivantes :
a. la plaque à buses (90) présente une plaque de base (94) en un matériau qui ne contient pas d'argent ou de matériau contenant de l'argent,
b. une couche (96 ; 98) en argent ou en un matériau contenant de l'argent est prévue sur la plaque de base (90),
de préférence comprenant l'une des caractéristiques supplémentaires suivantes :
c. la couche (96 ; 98) en argent ou en argent ou en un matériau contenant de l'argent est appliquée sur la plaque de base (94) par vaporisation ou immersion après la réalisation des ouvertures de buses (92), de telle sorte que les ouvertures de buses (92) soient au moins en partie recouvertes avec cette couche au niveau de leurs côtés intérieurs, ou
d. les ouvertures de buses (92) sont réalisées après la vaporisation ou l'immersion de la plaque de base (94) afin de fabriquer la couche d'argent ou d'oxyde d'argent, de telle sorte que les ouvertures de buses (92) soient au moins en partie exemptes de cette couche au niveau de leur côté intérieur.

12. Dispositif d'inhalation (10) selon l'une quelconque des revendications 3 à 11, comprenant la caractéristique suivante :
a. le réservoir de liquide est rempli d'un liquide choisi parmi le groupe comprenant les liquides suivants :
- une solution aqueuse salée,
- une solution aqueuse sous la forme d'un liquide de Ringer ou d'une solution tamponnée,
- une solution aqueuse comprenant au moins l'un des additifs hydrates de carbone, huiles essentielles, menthol et extraits végétaux,
- une solution aqueuse contenant des vitamines, des oligo-éléments, du manganèse ou du zinc, ou
- une solution aqueuse comprenant au moins l'un des additifs du groupe comprenant de l'huile de cannelle, de l'huile d'arbre à thé, de l'huile de sauge, de l'huile de thym, de l'huile de mélisse.

13. Jeu de dispositif d'inhalation comprenant les caractéristiques suivantes :
a. le jeu comprend un dispositif d'inhalation (10) selon l'une quelconque des revendications précédentes 3 à 12, et
b. le jeu comprend au moins deux unités structurelles (70) qui comprennent chacune une pièce d'applicateur (80 ; 86) et une plaque à buses (90).
